# EUROPEAN PATENT SPECIFICATION

(11) **EP 1 515 734 B1**
(45) Date of publication and mention of the grant of the patent: **08.08.2012**
(21) Application number: 03760969.0
(22) Date of filing: 19.06.2003
(51) Int. Cl.: A61K 35/64, A61K 47/48, A61K 47/32, A61L 15/24, A61K 9/00, A61P 17/00, A61P 31/04

(54) **HONEY-BASED SKIN CARE PREPARATION**
HAUTPFLEGEPRÄPARAT AUF HONIGBASIS
PREPARATION POUR LE SOIN DE LA PEAU A BASE DE MIEL

(30) Priority: 21.06.2002 BE 200200405
(43) Date of publication of application: 23.03.2005
(73) Proprietor: Triticum Patent & Trademark GmbH, 6304 Zug (CH)
(72) Inventor: VANDEPUTTE, Jan, B-8490 Varsenare (BE)
(74) Representative: Dohmen, Johannes Maria Gerardus
(86) International application number: PCT/NL2003/000451
(87) International publication number: WO 2004/000339

(56) References cited:
- EP-A- 0 503 853
- WO-A-02/30467
- US-A- 4 671 267
- US-A- 5 980 875
- US-B1- 6 174 535
- J. VANDEPUTTE: "Bee's best skin care products - Scientific information" ONLINE, August 2000 (2000-08), pages 1-9, XP002169765 Retrieved from the Internet: <URL:http://www.triticum.nl/english/s_arti cles/skin_care.htm> [retrieved on 2001-06-14]

## Description

The invention relates to a composition comprising honey as a therapeutically active compound for use in the treatment of wounds, wherein said composition is an aqueous polymeric gel based on acrylic monomers.

Concerning skin care preparations, a wound dressing is known from US 4,671,267 wherein a polymer matrix containing a water soluble humectant is used. Said document especially emphasizes the physiotherapeutic possibilities of the dressing, such as the "hot or cold" treatment. No mention is made therein of active or therapeutical ingredients. The term humectant is understood to mean a product that attracts moisture.

The drawback of such a skin care preparation is that the wound dressing effects a limited antibacterial activity. Such a wound dressing does not contain an active ingredient and it has no nutritional value for the wound.

From US patent No. 5,980,875 there is known a method for the preparation of a honey-based formulation for topical application, comprising the forming of a base as a carrier and the mixing of active constituents of honey with the base, with the mixing of the active constituents further comprising the setting of a mixing temperature not exceeding 40 °C. Although said US patent specification makes mention of the fact that a thickening agent may be added as a hydrophylic component, no further details with regard to the polymer gel are known therefrom.

From US patent No. 6,174,535 there is known a hydrated honey gel polymeric composition, which composition comprises: polyglycerylmethacrylate and a liquid polysaccharide component selected from the group consisting of honey and corn syrup, wherein the weight ratio of the polyglycerylmethacrylate to the polysaccharide in the liquid polysaccharide component is from about 8:1 to about 1:2.

International patent application WO 02/30467 discloses the use of a honey-based composition as a therapeutically active ingredient, which composition furthermore comprises lanolin and/or a lanolin derivative, which is in particular suitable for treating wounds. The use of a polymeric gel is not known therefrom.

Concerning honey-containing skin care preparations, a composition comprising a therapeutically active compound with antiseptic, osmotic and other characteristics is known from US 5,785,972, which composition is used in particular for the treatment of burns and open wounds experienced by animals and man, and in particular for the treatment of thermal burns on humans by use of spray composition. The composition comprises a combination of colloidal silver, helichrysum oil and raw honey emulsified with an emulsifier so as to form a solution, wherein water soluble lecithin is used as the emulsifier.

EP 0 503 853 discloses a method for preparing a composition for topical application, said composition comprising units derived from acrylamide, 2-acrylamido-2-methyl-propane-sulphonic acid and a polyfunctional monomer.

An article from J. Vandeputte "Bees' best skin care products - Scientific information" discloses the chemical and physical properties of honey and its positive influence on wound healing.

The drawback of such a skin care preparation is that a spray or a complex ointment will not remain present on a wound and will have to be covered with a secondary dressing at all times. As a result, the absorption of moisture does not take place directly from the wound.

A first aspect of the present invention is to provide a skin care preparation that does not exhibit the drawbacks as mentioned above.

A second aspect of the present invention is to provide a skin care preparation based on honey as a therapeutically active compound, which preparation is suitable for treating wounds, such as first and second degree burns, open wounds (chronic wounds), diabetic foot sores, bedsores (decubitus) and other skin surface wounds.

Yet another aspect of the present invention is to provide a skin care preparation having a special consistency or cohesion, wherein the honey as the active component can demonstrate the therapeutic effect thereof for a prolonged period of time, with a constant performance being ensured.

Another aspect of the present invention is to provide a skin care preparation which, when used on the human skin, will not stick to the skin tissue that forms the wound or to the surrounding skin tissue.

The present invention is characterized by the characterizing portion of claim 1.

One or more of the aforesaid aspects are accomplished by using such a special polymeric gel. It has in particular been found that the present polymeric gel exhibits a very good moisture absorption capacity and a very stable cohesion without any dehydration or cracking of the polymeric gel, which might lead to additional wounds and irritation of the patient's skin.

Preferably, the skin care preparation according to the invention is suitable for healing wounds on the one hand and for cosmetic use on the other hand.

A skin care preparation not according to the invention comprises
- from 5 to 60 wt.% honey, and
- from 30 to 40 wt.% polymeric gel

* including 10-30 wt.% polymer, and
* 10-30 wt.% water,
based on the total amount of ingredients.

The skin care preparation according to the invention, comprises a humectant in addition to honey and a polymeric gel.

The advantage of this is that it enhances the moisture absorption characteristics of the polymeric gel with honey entrapped therein.

The skin care preparation according to the invention comprises
- from 5 to 60 wt.% humectant,
- from 5 to 60 wt.% honey, and
- from 30 to 40 wt.% polymeric gel

* including 10-30 wt.% polymer, and
* 10-30 wt.% water,
based on the total amount of ingredients.

In one preferred embodiment of a skin care preparation according to the invention, said humectant is glycerin.

The advantage of this is that besides the honey as the therapeutically active compound, glycerin has a synergetic effect as regards the antibacterial activity of the skin care preparation.

In order to obtain an optimum functioning of the present skin care preparation, the amount of honey preferably ranges in particular from 10 to 35%, based on the total amount of ingredients. Such an amount of honey, in combination with the special polymeric gel, provides a skin care preparation which is pleasant and soft to the touch, and which moreover exhibits a very quick absorption of moisture from the wound, which polymer does not disintegrate, not even after absorption of moisture.

In a very suitable skin care preparation, one or more monomers comprising 2-acrylamido-2-methylpropane sulphonic acid, acrylic acid, acrylic acid (3-sulphopropyl)ester, a substituted derivative thereof or a salt thereof are preferably used as acrylic monomers.

In such an embodiment, one or more monomers comprising at least one of acrylamide or a mono- or di-N-alkylacrylamide or an analog compound thereof containing an alkyl or a substituted alkyl group combined with a carbon-carbon double binding via an amido- or alkylamido function are used as acrylic monomers.

Preferred analog compounds are diacetone acrylamide, a vinyllactam, an N-alkylated acrylamide, an N,N-dialkylated acrylamide, N-vinylpyrrolidone or acryloylmorpholin.

The preparation of such a polymer is known to the skilled men in the art and need not be explained in more detail therein, therefore. It stands to reason that the preparation of such a polymer comprises the addition of a photo initiator, which is capable of generating free radicals, a cross-linking agent and water besides the use of the active monomer components, followed by the treatment of the mixture in such a manner, for example by irradiating it with light of sufficient intensity and the desired wavelength, that polymerisation and crosslinking of the mixture will take place. If desired, one or more other substances can be added to the mixture to be polymerised, for example softeners, colorants, stabilisers, surfactants and preservatives.

The polymeric gel of the skin preparation according to the invention preferably comprises 50 wt. % acrylamide and 50 wt. % water, based on the total amount of ingredients in the polymeric gel.

In one preferred skin care preparation according to the invention, said skin care preparation comprises one or more additional ingredients selected from the group of antioxidants, transretinoic acid and/or derivatives and precursors thereof, polyunsaturated fatty acids, n-hexacosanol, bis(maltolato)oxovanadium(IV), aloë vera or thickeners.

In a specific preferred skin care preparation according to the invention, the amount of additional components constitutes up to 40 wt.% of the total amount of ingredients. In the

In the skin care preparation according to the invention the honey has a peroxide number of more than 5 µg/g honey/hour, measured at a temperature of 21 °C.

If the skin care preparation according to the invention is used for healing wounds, the honey is preferably subjected to a sterilisation treatment.

Preferably, said sterilisation treatment is carried out by means of gamma rays.

In one preferred embodiment of the skin care preparation according to the invention, the skin care preparation is in the form of a supple gel plate.

A skin care preparation according to the invention consists of a polymeric gel with honey being entrapped therein. This skin care preparation is quite suitable for healing acute wounds as well as chronic wounds, but it may also be used for cosmetic purposes.

The polymeric gel preferably consists of acrylic monomers, more specifically acrylamide and water in a proportion of 50/50 wt. %, based on the total amount of ingredients in the polymeric gel. The thickness of the polymeric gel may vary from 0.3 cm to 1.5 cm, in specific embodiments even from 0.2 to 2 mm.

Honey has a number of specific characteristics, such as being:
- hyperosmolar;
- trophic (= nutritional);
- antibacterial;
- debriding (in the case of scabbing of a wound, the scab will come loose and/or dissolve).

In addition to honey, the polymeric gel comprises a humectant. A humectant is a substance that attracts moisture. The following products may be used as humectants: glycerin, polyethyleneglycol, dimethylsulphoxide and dimethylformamide, etc. A humectant is a substance which has a strong softening effect, which, on subjective evaluation, makes skin which is dry or rough due to an inadequate natural moisture retention, feel softer and better.

Preferably, glycerin is used. Glycerin thus contributes towards maintaining or restoring the required moisture level in the skin. Glycerin has a synergetic effect with honey as regards the antibacterial activity. Glycerin furthermore ensures that the suppleness of the polymeric gel is not lost.

In that case, the skin care preparation comprises:
- from 5 to 60 wt.% humectant,
- from 5 to 60 wt.% honey, and
- from 30 to 40 wt.% polymeric gel

* including 10-30 wt.% polymer, and
* 10-30 wt.% water,
based on the total amount of ingredients.

The polymeric gel in combination with the honey and the glycerin provides an exchange system that works on the basis of gradient differences at a semi-permeable membrane. Honey and glycerin are more specifically hyperosmolar substances. The exchange system works as follows: moisture from the skin surface to be treated migrates towards the polymeric gel, whilst the honey and the glycerin migrates towards the skin surface to be treated. The antibacterial agent, in particular honey, is thus gradually released, whilst in addition excess wound moisture is absorbed in the polymeric gel.

In the cosmetic sphere, the skin preparation according to the invention acts on the epidermis of the skin. The glycerin effects a normalisation of the moisture level in the epidermis, whilst the honey removes free oxygen radicals via H₂0₂.

The skin care preparation preferably comprises one or more additional components selected from the group of antioxidants, transretinoic acid and/or derivatives and precursors therefrom, polyunsaturated fatty acids, n-hexacosanol, bis(maltolato)oxovanadium(IV), aloë vera or thickeners. Examples of thickeners are carboxymethyl cellulose or polyethylene glycol.

Especially preferred is vitamin A or a derivative thereof, for example a vitamin A-containing composition such as cod-liver oil. Vitamin A can be considered to be a precursor of vitamin A acid and has a positive effect on the healing process of wounds. The addition thereof is in particular desirable in the case of a retarded healing process of the patient, for example caused by the administration of corticosteroids. It is assumed that vitamin A or a derivative thereof stimulates the formation of type I and type III collagen and incites the epidermis cells to divide in an orderly manner.

Preferably, the skin care preparation comprises up to 40 wt. % additional ingredients, based on the total amount of ingredients.

The honey that is used in the skin care preparation according to the invention must have one or more special characteristics.

The honey must have a peroxide number of more than 5 µg/g honey/hour, measured at a temperature of 21 °C. If honey having a peroxide count outside the aforesaid range is used, the antibacterial activity of the honey will be insufficient.

Since the skin care preparation may be directly applied to the wound, it must furthermore be sterile. This can be effected by sterilising the entire amount of polymeric gel by means of gamma rays, for example gamma rays obtained from cobalt 60. Experiments have shown that the honey retains its antibacterial activity after being subjected to such radiation.

The honey must be free from heavy metals, pesticides and herbicides. These requirements are made because the skin care preparation is directly applied to the wound.

The following procedure is followed for preparing the skin care preparation:
- the honey, the humectant and any additional ingredients are added to the liquid polymer;
- the aggregate is mixed, preferably by means of a mixer;
- a catalyst that initiates the chain reaction is added;
- after activation, for example by irradiation with UV light having the desired wavelength or by heating, depending on the reaction system, the chain reaction is started within a few seconds to a few minutes, resulting in the forming of the polymeric gel.

The present invention will now be explained in more detail by means of a number of examples. It will be understood that no aspect of the following description is to be interpreted as limiting the scope of the invention as defined in the appending claims.

### Example 1

### - Comparative example:

A person suffering from a severe case of decubitus was treated with pure honey, which was applied to the wound site. Although the healing process of the bedsores showed a slight improvement in comparison with the situation in which no honey was applied, the patient experienced undesirable pain. Part of the honey is quickly lost outside the wound area. Extra moisture being extracted from the wound runs off and must be absorbed by a secondary dressing.

### - Example of the use of a skin care preparation according to the invention:

A composition consisting of 20 wt.% glycerin and 45 wt.% honey and 17.5% polyacryl matrix was used on a group of patients suffering from decubitus. The bedsores healed remarkably quickly, whilst in addition the patients did not experience the pain that was experienced in Comparative example 1. The dressing could remain in place longer because the release of the honey to the wound takes place gradually.

### Example 2

### - Example of the use of a skin care preparation according to the invention:

The composition of Example 1 was used, with this difference that additionally 10 wt.% cod-liver oil was incorporated therein. Patients treated with this composition experienced a very quick healing of their open wounds, whilst in addition their skin fully regained its smooth surface structure.

### - Comparative example 2:

A composition consisting of 17.5 wt.% acrylamide matrix and 65 wt.% glycerin was used with patients suffering from deep grazes/burns. Although this composition exhibited some therapeutic activity, the time it took for the wounds to heal was considerably longer than in the situation in which a composition that contained honey besides glycerin was used.

### Example 3

### - Example of the use of a skin care preparation according to the invention:

A composition consisting of 30 wt.% glycerin and 35 wt.% honey was used on a group of patients suffering from deep grazes/burns. The composition was experienced as pleasant by the patients and the wounds healed within two weeks.

## Claims

1. A composition comprising honey as a therapeutically active compound for use in the treatment of wounds, wherein said composition is an aqueous polymeric gel based on acrylic monomers, **characterized in that** said composition comprises
- from 5 to 60 wt.% humectant,
- from 5 to 60 wt.% honey, and
- from 30 to 40 wt.% polymeric gel
* including 10-30 wt.% polymer, and
* 10-30 wt.% water,
based on the total amount of ingredients, wherein the honey has a peroxide number of more than 5 µg/g honey/hour, measured at a temperature of 21 °C.

2. A composition according to claim 1 for use in the treatment according to claim 1, **characterized in that** said humectant is glycerin.

3. A composition according to any one or more of the preceding claims for use in the treatment according to claim 1, **characterized in that** the amount of honey ranges from 10 to 35%, based on the total amount of ingredients.

4. A composition according to any one or more of the claims 1 - 3 for use in the treatment according to claim 1, **characterized in that** one or more monomers comprising 2-acrylamido-2-methylpropane sulphonic acid, acrylic acid, acrylic acid (3-sulphopropyl)ester, a substituted derivative thereof or a salt thereof are used as acrylic monomers.

5. A composition according to any one or more of the claims 1-4 for use in the treatment according to claim 1, **characterized in that** one or more monomers comprising at least one of acrylamide or a mono- or di-N-alkylacrylamide or an analog compound thereof containing an alkyl or a substituted alkyl group combined with a carbon-carbon double binding via an amido- or alkylamido function are used as acrylic monomers.

6. A composition according to claim 5 for use in the treatment according to claim 1, **characterized in that** the analog compound is diacetone acrylamide, a vinyllactam, an N-alkylated acrylamide, an N,N-dialkylated acrylamide, N-vinylpyrrolidone or acryloylmorpholin.

7. A composition according to any one or more of the claims 1-6 for use in the treatment according to claim 1, **characterized in that** the polymeric gel comprises 50 wt. % acrylamide and 50 wt. % water, based on the total amount of ingredients in the polymeric gel.

8. A composition according to any one or more of the claims 1-7 for use in the treatment according to claim 1, **characterized in that** said skin care preparation comprises one or more additional ingredients selected from the group of antioxidants, transretinoic acid and/or derivatives and precursors thereof, polyunsaturated fatty acids, n-hexacosanol, bis(maltolato)oxo-vanadium(IV), aloë vera or thickeners.

9. A composition according to claim 8 for use in the treatment according to claim 1, **characterized in that** the amount of additional components forms up to 40 wt.% of the total amount of ingredients.

10. A composition according to any one or more of the preceding claims for use in the treatment according to claim 1, **characterized in that** the honey is subjected to a sterilisation treatment when used for healing wounds.

11. A composition according to claim 10 for use in the treatment according to claim 1, **characterized in that** said sterilisation treatment is carried out by means of gamma rays.

12. A composition according to any one or more of the preceding claims for use in the treatment according to claim 1, **characterized in that** said skin care preparation takes the form of a supple gel plate having a thickness of 0.2-2 mm.

## Patentansprüche

1. Zusammensetzung, die Honig als therapeutisch aktive Verbindung zur Verwendung bei der Behandlung von Wunden enthält, wobei die Zusammensetzung ein wässriges Polymergel auf der Grundlage von Acrylmonomeren ist, **dadurch gekennzeichnet, dass** die Zusammensetzung umfasst:
- von 5 bis 60 Gew.-% Feuchthaltemittel,
- von 5 bis 60 Gew.-% Honig, und
- von 30 bis 40 Gew.-% Polymergel
* enthaltend 10 - 30 Gew.-% Polymer, und
* 10 - 30 Gew.-% Wasser,
basierend auf der Gesamtmenge an Inhaltsstoffen, wobei der Honig eine Peroxidzahl von über 5µg/g Honig/Stunde hat, gemessen bei einer Temperatur von 21°C.

2. Zusammensetzung nach Anspruch 1 zur Verwendung bei der Behandlung nach Anspruch 1, **dadurch gekennzeichnet, dass** das Feuchthaltemittel Glyzerin ist.

3. Zusammensetzung nach einem oder mehreren der vorhergehenden Ansprüche zur Verwendung bei der Behandlung nach Anspruch 1, **dadurch gekennzeichnet, dass** die Menge an Honig im Bereich von 10 bis 35% basierend auf der Gesamtmenge an Inhaltsstoffen rangiert.

4. Zusammensetzung nach einem oder mehreren der Ansprüche 1 bis 3 zur Verwendung bei der Behandlung nach Anspruch 1, **dadurch gekennzeichnet, dass** ein oder mehrere Monomere umfassend 2-Acrylamido-2-Methylpropan-Sulfonsäure, Acrylsäure, Acrylsäure-(3-Sulfopropyl)-Ester, ein substituiertes Derivat hiervon oder ein Salz hiervon als Acrylmonomere verwendet werden.

5. Zusammensetzung nach einem oder mehreren der Ansprüche 1 bis 4 zur Verwendung bei der Behandlung nach Anspruch 1, **dadurch gekennzeichnet, dass** als Acrylmonomere ein oder mehrere Monomere verwendet werden, die Acrylamid und/oder ein Mono- oder di-N-Alkylacrylamid oder eine hierzu analoge Verbindung umfassen, die eine Alkylgruppe oder eine substituierte Alkylgruppe umfasst, die über eine Amido- oder Alkylamido-Funktion mittels einer Kohlenstoff/Kohlenstoff-Doppelbindung gebunden ist.

6. Zusammensetzung nach Anspruch 5 zur Verwendung bei der Behandlung nach Anspruch 1, **dadurch gekennzeichnet, dass** die analoge Verbindung Diacetonacrylamid, ein Vinyllactam, ein N-alkyliertes Acrylamid, ein N,N-dialkyliertes Acrylamid, N-Vinylpyrrolidon oder Acryloylmorpholin ist.

7. Zusammensetzung nach einem oder mehreren der Ansprüche 1 bis 6 zur Verwendung bei der Behandlung nach Anspruch 1, **dadurch gekennzeichnet, dass** das Polymergel 50 Gew.-% Acrylamid und 50 Gew.-% Wasser enthält, basierend auf der Gesamtmenge an Inhaltsstoffen im Polymergel.

8. Zusammensetzung nach einem oder mehreren der Ansprüche 1 bis 7 zur Verwendung bei der Behandlung nach Anspruch 1, **dadurch gekennzeichnet, dass** das Hautpflegepräparat einen oder mehrere zusätzliche Inhaltsstoffe umfasst, die aus der Gruppe aus Antioxidantien, trans-Retinsäure und/oder Derivaten und Vorläuferstoffen hiervon, mehrfach ungesättigten Fettsäuren, n-Hexacosanol, bis(maltolato)oxo-Vanadium(IV), Aloe Vera oder Verdickungsmitteln ausgewählt sind.

9. Zusammensetzung nach Anspruch 8 zur Verwendung bei der Behandlung nach Anspruch 1, **dadurch gekennzeichnet, dass** die Menge an zusätzlichen Bestandteilen bis zu 40 Gew.-% der Gesamtmenge an Inhaltstoffen darstellt.

10. Zusammensetzung nach einem oder mehreren der vorhergehenden Ansprüche zur Verwendung bei der Behandlung nach Anspruch 1, **dadurch gekennzeichnet, dass** der Honig einer Sterilisationsbehandlung unterzogen wird, wenn sie zur Heilung von Wunden verwendet wird.

11. Zusammensetzung nach Anspruch 10 zur Verwendung bei der Behandlung nach Anspruch 1, **dadurch gekennzeichnet, dass** die Sterilisationsbehandlung mittels Gammastrahlen ausgeführt wird.

12. Zusammensetzung nach einem oder mehreren der vorhergehenden Ansprüche zur Verwendung bei der Behandlung nach Anspruch 1, **dadurch gekennzeichnet, dass** das Hautpflegepräparat die Form einer weichen Gelplatte mit einer Dicke von 0,2 bis 2 mm annimmt.

## Revendications

1. Composition comprenant du miel comme composé thérapeutiquement actif à utiliser dans le traitement de plaies, dans laquelle ladite composition est un gel polymérique aqueux à base de monomères acryliques, **caractérisée en ce que** ladite composition comprend
- de 5 à 60 % en poids d'humectant,
- de 5 à 60 % en poids de miel, et
- de 30 à 40 % en poids de gel polymérique
-- comprenant de 10 à 30 % en poids de polymère, et
-- de 10 à 30 % en poids d'eau,
d'après la quantité totale d'ingrédients, dans laquelle le miel a un indice de peroxyde de plus de 5 µg/g de miel/heure, mesuré à une température de 21 °C.

2. Composition selon la revendication 1, à utiliser dans le traitement selon la revendication 1, **caractérisé en ce que** l'humectant est la glycérine.

3. Composition selon l'une quelconque ou plusieurs des revendications précédentes à utiliser dans le traitement selon la revendication 1, **caractérisée en ce que** la quantité de miel varie de 10 à 35 %, d'après la quantité totale d'ingrédients.

4. Composition selon l'une quelconque des revendications 1 à 3 à utiliser dans le traitement selon la revendication 1, **caractérisée en ce qu'**un ou plusieurs monomères comprenant de l'acide 2-acrylamido-2-méthylpropane sulfonique, de l'acide acrylique, de l'acide acrylique de (3-sulfopropyl)ester, un dérivé substitué de celui-ci ou un sel- de celui-ci sont utilisés comme monomères acryliques.

5. Composition selon l'une quelconque ou plusieurs des revendications 1 à 4 à utiliser dans le traitement selon la revendication 1, **caractérisée en ce qu'**un ou plusieurs monomères comprenant au moins l'un d'un acrylamide ou d'un mono- ou di-N-alkylacrylamide ou d'un composé analogue de celui-ci contenant un alkyle ou un groupe alkyle substitué combiné avec une double liaison carbone-carbone via une fonction amido ou alkylamido sont utilisés comme monomères acryliques.

6. Composition selon la revendication 5 à utiliser dans le traitement selon la revendication 1, **caractérisée en ce que** le composé analogue est le diacétone acrylamide, un vinyl lactame, un acrylamide N-alkylé, un acrylamide N,N-dialkylé, de la N-vinylpyrrolidone ou de l'acryloylmorpholine.

7. Composition selon l'une quelconque ou plusieurs des revendications 1 à 6 à utiliser dans le traitement selon la revendication 1, **caractérisée en ce que** le gel polymérique comprend 50 % en poids d'acrylamide et 50 % en poids d'eau, d'après la quantité totale d'ingrédients dans le gel polymérique.

8. Composition selon l'une quelconque ou plusieurs des revendications 1 à 7 à utiliser dans le traitement selon la revendication 1, **caractérisée en ce que** ladite préparation de soin pour la peau contient un ou plusieurs ingrédients supplémentaires choisis dans le groupe des antioxydants, de l'acide transrétinoïque et/ou des dérivés et précurseurs de celui-ci, acides gras polyinsaturés, n-hexacosanol, vanadium bis(maltolato)oxo (IV), aloé vera ou épaississants.

9. Composition selon la revendication 8 à utiliser dans le traitement selon la revendication 1, **caractérisée en ce que** la quantité de composants supplémentaires forme jusqu'à 40 % en poids de la quantité totale d'ingrédients.

10. Composition selon l'une quelconque ou plusieurs des revendications précédentes à utiliser dans le traitement selon la revendication 1, **caractérisée en ce que** le miel est soumis à un traitement de stérilisation lorsqu'il est utilisé pour guérir les plaies.

11. Composition selon la revendication 10 à utiliser dans le traitement selon la revendication 1, **caractérisée en ce que** ledit traitement de stérilisation est réalisé au moyen de rayons gamma.

12. Composition selon l'une quelconque ou plusieurs des revendications précédentes à utiliser dans le traitement selon la revendication 1, **caractérisée en ce que** ladite préparation de soin pour la peau prend la forme d'une plaque de gel souple ayant une épaisseur de 0,2 à 2 mm.
